# FASCICULE DE BREVET EUROPEEN

(11) **EP 1 655 290 B1**
(45) Date de publication et mention de la délivrance du brevet: **29.04.2009**
(21) Numéro de dépôt: 05292132.7
(22) Date de dépôt: 12.10.2005
(51) Int. Cl.: C07D 275/06, C07D 275/04, A61K 31/428

(54) **Compositions, notamment cosmétiques, comprenant une benzoisothiazolone**
ZUSAMMENSETZUNGEN ENTHALTEND BENZOISOTHIAZOLDERIVATE UND IHRE VERWENDUNG IM KOSMETISCHEN BEREICH
Compositions comprising a benzoisothiazolone derivative and their use in the cosmetic field

(30) Priorité: 08.11.2004 FR 0452563
(43) Date de publication de la demande: 10.05.2006
(73) Titulaire: L'ORÉAL, 75008 Paris (FR)
(72) Inventeur: Lucet-Levannier, Karine, 92500 Reuil-Malmaison (FR); Cavezza, Alexandre, 93320 Pavillon Sous Bois (FR); Erdelmeier, Irène, 75013 Paris (FR)
(74) Mandataire: Kromer, Christophe

(56) Documents cités:
- EP-A- 0 644 243
- EP-A- 1 442 737
- US-A- 4 654 354

## Description

La présente invention concerne une composition, adaptée à une application topique sur la peau, comprenant au moins une benzoisothiazolone de formule donnée, ainsi qu'un procédé cosmétique de soin d'une peau sèche et/ou mature, comprenant l'application topique sur ladite peau de la composition précitée.

De nombreuses femmes à partir de trente-cinq ans, et plus particulièrement après la ménopause, se plaignent fréquemment du dessèchement de leur peau, et des manifestations d'inconfort ou inesthétiques qui en résultent (desquamation, teint terne, atonie cutanée). Or, ce dessèchement est dû, comme on le sait maintenant, à une diminution de la production de sébum avec l'âge.

Le sébum est le produit naturel de la glande sébacée qui, conjointement à la sueur produite par les glandes eccrines ou aprocrines, constitue un hydratant naturel de l'épiderme. Il est constitué essentiellement d'un mélange plus ou moins complexe de lipides. Classiquement, la glande sébacée produit du squalène, des triglycérides, des cires aliphatiques, des cires de cholestérol et éventuellement du cholestérol libre (Stewart, M. E., Semin. Dermatol. 11, 100-105 (1992)). L'action des lipases bactériennes convertit une part variable des triglycérides en acides gras libres.

Le sébocyte constitue la cellule compétente de la glande sébacée. La production de sébum est associée au programme de différenciation terminale de cette cellule. Durant cette différenciation, l'activité métabolique du sébocyte est essentiellement axée sur la biosynthèse des lipides (la lipogénèse) et plus précisément sur la néosyntèse d'acides gras et du squalène.

Un composé permettant de stimuler la production des lipides constituant le sébum, par les cellules de la glande sébacée (les sébocytes), serait donc d'un intérêt certain pour le traitement des peaux sèches oligoséborrhéiques, c'est-à-dire présentant un taux de sébum inférieur à 100 µg/cm² au niveau du front.

A cette fin, il a été proposé dans le brevet US-4,496,556 d'utiliser la DHEA, un stéroïde sécrété par les glandes surrénales, ou ses esters, administrés par voie topique, pour augmenter la production de sébum.

Toutefois, pour des questions réglementaires, il n'est pas toujours possible d'utiliser ce type de composés dans le domaine cosmétique. En outre, son efficacité n'est pas suffisante sur les peaux oligoséborrhéiques. Il subsiste donc le besoin de disposer de composés cosmétiquement acceptables, permettant de stimuler efficacement la fonction sébacée en vue de traiter les peaux sèches oligoséborrhéiques.

De tels composés seraient également utiles dans le traitement des cuirs chevelus secs qui sont souvent associés à des cheveux ternes et atones.

La demanderesse a maintenant découvert avec étonnement que certaines benzoisothiazolones permettaient de satisfaire ce besoin.

Les benzoisothiazolones objets de la présente invention font partie d'une classe de composés qui ont déjà été décrits comme agents destinés à protéger un milieu d'enregistrement optique contre l'humidité (JP-10 027 381), comme stabilisants dans la fabrication de moules en polycarbonate, en vue de conférer à ces moules une tenue au rayonnement et à la vapeur surchauffée (EP-0 742 260), ou encore comme agents anti-cancéreux (JP-04 077 476).

Des benzoisothiazolones proches de celles objets de cette invention ont par eilleurs été décrites comme agents anti-bactériens et anti-fongiques, par exemple dans le traitement local des dermatophytoses (US-3,012,039).

Certaines benzoisothiazolones couvertes par la présente invention sont par ailleurs connues pour empêcher l'adhésion des organismes aquatiques nuisibles sur des surfaces (par exemple de bateaux ou d'usines) exposées à l'eau (EP-0 644 243).

Enfin, la 1,1-dioxyde N-(5-méthoxy-2-phényl)-1,2-benzoisothiazolone fait partie d'une librairie chimique (n° CAS : 632300-86-8).

Ainsi, il n'a jamais été décrit de composition, notamment cosmétique, adaptée à une application topique sur la peau, comprenant les benzoisothiazolones selon l'invention.

La présente invention a donc pour objet une composition, adaptée à une application topique sur la peau, comprenant au moins un composé de formule (I) : 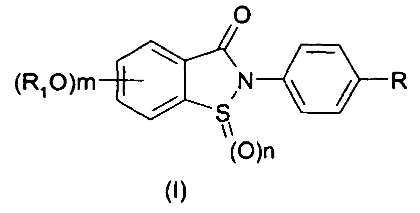 dans laquelle :
- R₁ est un atome d'hydrogène ou un radical méthyle,
- R est un atome d'hydrogène ou un radical méthoxy,
- m est égal à 1 ou 2,
- n est égal à 0, 1 ou 2.

Selon une forme d'exécution préférée de l'invention, m est égal à 1. Dans ce cas, le groupement OR₁ est de préférence situé en position 4 sur la benzoisothiazolone. Selon une autre forme d'exécution, m est égal à 2. Dans ce cas, les groupements OR₁ (identiques ou différents) sont de préférence situés en positions 5 et 7 sur la benzoisothiazolone.

Selon un mode de réalisation préféré, n est égal à 2.

Comme exemples de composés utilisables dans la présente invention, on peut citer :
- la 4-hydroxy-2-phényl-1,2-benzisothiazol-3(2H)-one
- le 4-hydroxy-2-phényl-1,2-benzisothiazol-3(2H)-one 1-oxide
- le 4-hydroxy-2-phényl-1,2-benzisothiazol-3(2H)-one 1,1-dioxide
- la 4-méthoxy-2-phényl-1,2-benzisothiazol-3(2H)-one
- le 4-méthoxy-2-phényl-1,2-benzisothiazol-3(2H)-one 1-oxide
- le 4-méthoxy-2-phényl-1,2-benzisothiazol-3(2H)-one 1,1-dioxide
- la 4-hydroxy-2-(4-méthoxyphényl)-1,2-benzisothiazol-3(2H)-one
- le 4-hydroxy-2-(4-méthoxyphényl)-1,2-benzisothiazol-3(2H)-one 1-oxide
- le 4-hydroxy-2-(4-méthoxyphényl)-1,2-benzisothiazol-3(2H)-one 1,1-dioxide
- la 4-méthoxy-2-(4-méthoxyphényl)-1,2-benzisothiazol-3(2H)-one
- le 4-méthoxy-2-(4-méthoxyphényl)-1,2-benzisothiazol-3(2H)-one 1-oxide
- le 4-méthoxy-2-(4-méthoxyphényl)-1,2-benzisothiazol-3(2H)-one 1,1-dioxide
- la 5,7-dihydroxy-2-phényl-1,2-benzisothiazol-3(2H)-one
- le 5,7-dihydroxy-2-phényl-1,2-benzisothiazol-3(2H)-one 1-oxide
- le 5,7-dihydroxy-2-phényl-1,2-benzisothiazol-3(2H)-one 1,1-dioxide
- la 5,7-diméthoxy-2-phényl-1,2-benzisothiazol-3(2H)-one
- le 5,7-diméthoxy-2-phényl-1,2-benzisothiazol-3(2H)-one 1-oxide
- le 5,7-diméthoxy-2-phényl-1,2-benzisothiazol-3(2H)-one 1,1-dioxide
- la 5,7-dihydroxy-2-(4-méthoxyphényl)-1,2-benzisothiazol-3(2H)-one
- le 5,7-dihydroxy-2-(4-méthoxyphényl)-1,2-benzisothiazol-3(2H)-one 1-oxide
- le 5,7-dihydroxy-2-(4-méthoxyphényl)-1,2-benzisothiazol-3(2H)-one 1,1-dioxide
- la 5,7-diméthoxy-2-(4-méthoxyphényl)-1,2-benzisothiazol-3(2H)-one
- le 5,7-diméthoxy-2-(4-méthoxyphényl)-1,2-benzisothiazol-3(2H)-one 1-oxide
- le 5,7-diméthoxy-2-(4-méthoxyphényl)-1,2-benzisothiazol-3(2H)-one 1,1-dioxide

Les composés préférés pour une utilisation dans la présente invention sont le 4-méthoxy-2-(4-méthoxyphényl)-1,2-benzisothiazol-3(2H)-one 1,1-dioxide de formule : 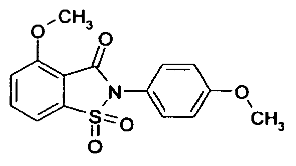 et le 4-hydroxy-2-phényl-1,2-benzisothiazol-3(2H)-one 1,1-dioxide de formule :

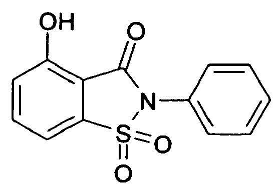

La présente invention a également pour objet de nouvelles benzoisothiazolones choisies parmi les composés de formule (I) ci-dessus à l'exception de ceux pour lesquels :
- n = 0 ; R = H ; m = 1 ; et R₁ = CH₃ ; et
- n = 2 ; R = H ; m = 1 ; et R₁ = CH₃.

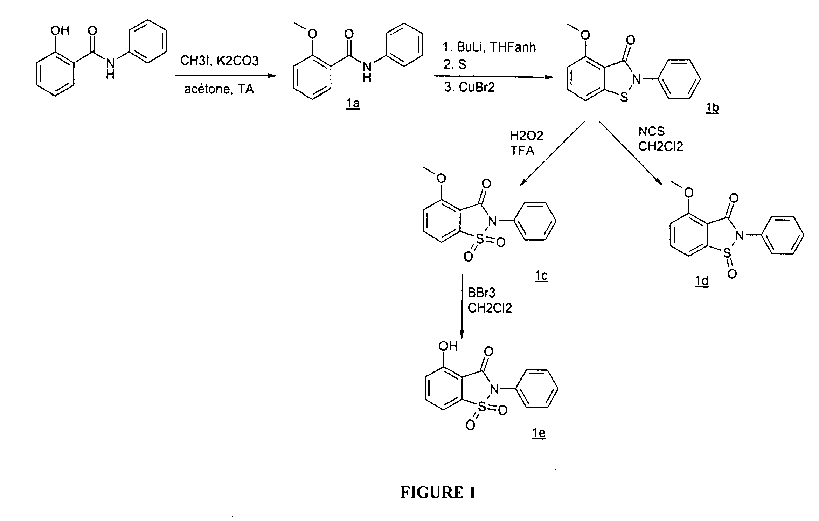

Les composés de formule (I) pour lesquels R = H, R₁ = CH₃ et m=1 peuvent être préparés suivant le schéma réactionnel illustré à la Figure 1 annexée, comme décrit aux Exemples 1 à 3 ci-après.

Les composés de formule (I) pour lesquels R = R₁ = H et m= 1 peuvent être préparés suivant le schéma réactionnel illustré à la Figure 1 annexée, comme décrit à l'Exemple 4 ci-après.

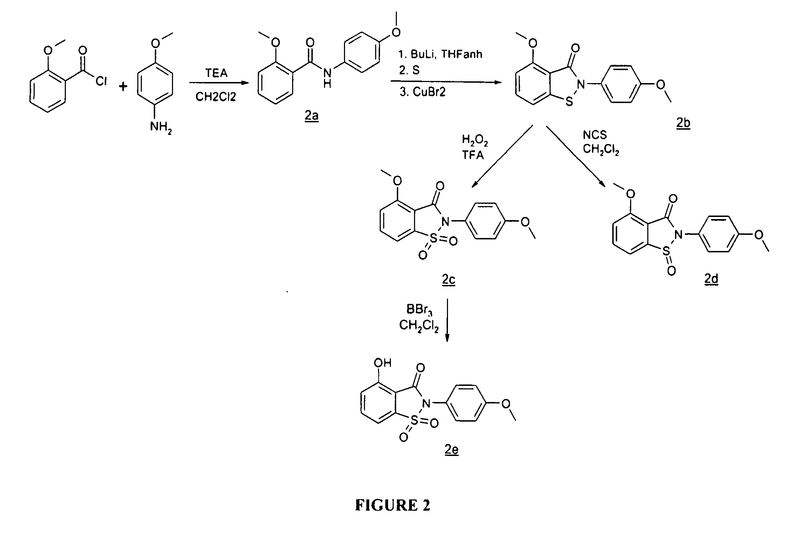

Les composés de formule (1) pour lesquels R = OCH₃, R₁ = CH₃ et m=1 peuvent être préparés suivant le schéma réactionnel illustré à la Figure 2 annexée, comme décrit aux Exemples 5 à 7 ci-après.

Les composés de formule (I) pour lesquels R = OCH₃, R₁ = H et m = 1 peuvent être préparés suivant le schéma réactionnel illustré à la Figure 2 annexée, comme décrit à l'Exemple 8 ci-après.

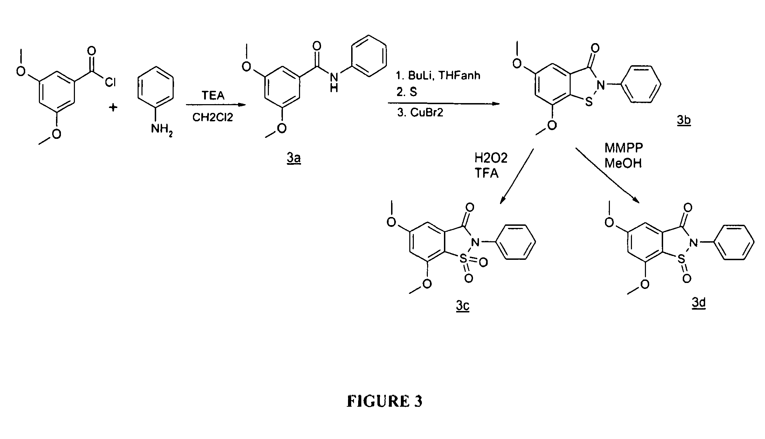

Enfin, les composés pour lesquels m = 2 peuvent être préparés suivant le schéma réactionnel illustré à la Figure 3 annexée, selon un procédé analogue à celui décrit aux Exemples 9 à 11 ci-après.

Les précurseurs N-phénylbenzamides mis en oeuvre dans ces procédés sont soit achetés, soit préparés classiquement à partir de la réaction du dérivé d'aniline correspondant sur le chlorure d'acyle correspondant en présence d'une base aminée telle que la triéthyle amine dans un solvant aprotique, comme par exemple le dichlorométhane.

L'étape d'introduction du soufre et de cyclisation est réalisée en une seule étape, selon le mode opératoire d'écrit dans la publication : JOC, 1989, 54, 2964-2966 .

Les oxydations des benzoisothiazolones sont réalisés par des réactifs classiques d'oxydation du soufre, à savoir H₂O₂ / acide trifluoroacétique (TFA) pour les dioxides et monoperoxyphtalate de magnésium hexahydrate (MMPP) ou N-chlorosuccinimide (NCS) pour les oxides. Ces méthodes sont bien connues pour l'homme de l'art et décrites notamment dans JOC. 2000, 65, 8439-8443 ; JOC. 2000, 65, 6462-6473 et Bull. Chem. Soc. Jpn, 58, 3131 (1985).

La Demanderesse a démontré que la composition renfermant les composés de formule (I) permettait de stimuler significativement la production des lipides constituant le sébum par les cellules de la glande sébacée. Cette composition trouve donc un intérêt dans le soin des peaux sèches, ou des cuirs chevelus secs, et plus particulièrement dans le soin des peaux matures. La zone de peau traitée par les composés selon l'invention est de préférence la peau du visage et/ou du cou et/ou des mains.

La présente invention a donc également pour objet un procédé cosmétique de soin d'une peau sèche et/ou mature, comprenant l'application topique sur la peau, en particulier du visage et/ou du cou et/ou des mains, de la composition définie précédemment.

La composition selon l'invention est particulièrement bien adaptée au traitement des peaux sèches oligoséborrhéiques, c'est-à-dire des peaux présentant un taux de sébum inférieur à 100 µg/cm² au niveau du front. Ce type de peau se rencontre fréquemment chez les femmes au voisinage de la ménopause, de sorte que la composition utilisée selon l'invention est de préférence appliquée sur des femmes de plus de quarante ans, de préférence de plus de cinquante ans, voire de plus de soixante ans.

La présente invention décrit l'utilisation cosmétique d'au moins un composé de formule (I), tel que défini précédemment, en tant qu'agent relipidant des peaux sèches ou matures.

Elle a également pour objet l'utilisation d'au moins un composé de formule (I), tel que défini précédemment, pour la préparation d'une composition, notamment dermatologique, destinée à traiter les désordres liés aux peaux sèches oligoséborrhéiques, en particulier les dermites.

La quantité de composé de formule (I) utilisable selon l'invention est bien entendu fonction de l'effet recherché et peut donc varier dans une large mesure. D'une manière générale, le composé de formule (I) sera présent en une quantité suffisante pour augmenter significativement la production de sébum, et avantageusement pour augmenter d'au moins 10% la production de sébum, par une culture de sébocytes, comme décrit dans l'Exemple 12 ci-après.

Pour donner un ordre de grandeur, on peut utiliser ce composé en une quantité représentant de 0,001 à 10 % du poids total de la composition, préférentiellement en une quantité représentant de 0,1 % à 5 % du poids total de la composition.

La composition selon l'invention est généralement adaptée à une application topique sur la peau (respectivement, le cuir chevelu) et elle contient donc un milieu physiologiquement acceptable, c'est-à-dire compatible avec la peau (respectivement, le cuir chevelu), et éventuellement avec ses phanères (cils, ongles, cheveux) et/ou les muqueuses. Ce milieu est avantageusement compatible avec la peau du visage et/ou du cou et/ou des mains. II s'agit de préférence d'un milieu cosmétiquement acceptable, c'est-à-dire qui ne génère pas d'inconfort (rougeurs, tiraillements, picotements) susceptibles de détourner l'utilisateur de la composition.

Pour une application topique sur la peau, cette composition peut se présenter sous toutes les formes galéniques normalement utilisées dans les domaines cosmétique et dermatologique, et elle peut être notamment sous forme d'une solution huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H). Ces compositions sont préparées selon les méthodes usuelles. On préfère utiliser selon cette invention une composition contenant de l'eau, susceptible de véhiculer des actifs hydrophiles, et en particulier une composition se présentant sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

Lorsqu'elle est adaptée et destinée à une application sur le cuir chevelu, la composition selon l'invention peut en variante être utilisée comme shampooing ou après-shampooing.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant peuvent être présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'amande d'abricot, huile d'olive, huile de soja), les huiles de synthèse, les huiles siliconées (cyclomethicone et dimethicones) et les huiles fluorées (perfluoropolyéthers). On peut aussi utiliser comme matières grasses des alcools gras (alcool cétylique), des acides gras, des cires (cire de carnauba, ozokérite).

Comme émulsionnants et co-émulsionnants utilisables dans l'invention, on peut citer par exemple les esters d'acide gras et de polyéthylène glycol tels que le stéarate de PEG-100, les esters d'acide gras et de glycérine tels que le stéarate de glycéryle, les esters de sucrose, les alkyl polyglycosides, les éthers d'alcools gras et de polyéthylène glycol, et les esters d'acide gras et de methyl glucose.

De façon connue, la composition selon l'invention peut contenir également les adjuvants habituels dans le domaine cosmétique, tels que les gélifiants / épaississants hydrophiles ou lipophiles, en particulier les homo- et copolymères d'acrylamide, les homo- et copolymères acryliques, les homo- et copolymères d'acide amcrylamido méthylproane sulfonique (AMPS) et les polysaccharides ; les actifs hydrophiles ou lipophiles ; les conservateurs ; les parfums ; les charges telles que la silice, les microsphères de polyamide et les fibres de polyamide ; les pigments ; et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse ou dans la phase aqueuse, le cas échéant. En tout état de cause, ces adjuvants, ainsi que leurs proportions, seront choisis de manière à ne pas nuire aux propriétés recherchées des composés de formule (I) utilisés selon l'invention.

Comme actifs, il sera avantageux d'introduire dans la composition utilisée selon l'invention au moins un composé choisi parmi : les agents desquamants ; les agents hydratants ; les agents apaisants ; et les agents stimulant la prolifération et/ou la différenciation des kératinocytes.

En effet, la stimulation de la séborrhée par les composés de formule (I) selon l'invention peut, chez certaines personnes, fournir un terrain de prolifération pour la microflore résidente de l'ostium folliculaire *(Propionibacterium acnes* en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut diverger vers une lésion acnéique inflammatoire.

L'ajout d'agents desquamants ou régulant la prolifération ou la différenciation des kératinocytes à la composition selon l'invention permettent d'éviter la formation de ces comédons. De même, des agents anti-bactériens ou bactériostatiques permettraient, en modérant la prolifération de la microflore résidente, d'obtenir le même effet.

En outre, les agents hydratants peuvent compléter l'effet obtenu à l'aide des composés selon l'invention, et les agents apaisants sont utiles pour améliorer le confort des peaux sèches oligoséborrhéiques.

Des exemples de tels actifs additionnels sont donnés ci-dessous.

### Agents desquamants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de *Saphora japonica ;* le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

### Agent hydratant

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA et ses dérivés, et la vitamine D et ses dérivés.

### Agents stimulant la prolifération et/ou la différenciation des kératinocytes

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de *Solanum tuberosum* commercialisés par la société SEDERMA.

Les rétinoïdes sont préférés pour une utilisation dans cette invention, en particulier le rétinol et ses esters.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

### Agents apaisants

Parmi les matières premières efficaces comme agents apaisants, on peut citer de façon non limitative les actifs suivants : les triterpènes pentacycliques, comme l'acide β-glycyrrhétinique et ses sels et/ou ses dérivés (acide glycyrrhétique monoglucuronide, stearyl glycyrrhetinate, acide 3- stéaroyloxy glycyrrhetique), l'acide ursolique et ses sels, l'acide oléanolique et ses sels, l'acide bétulinique et ses sels ; les extraits de *Paeonia suffruticosa* et/ou *lactiflora*, de *Rosmarinus officinalis*, d'épilobe, de *Pygeum,* de *Boswellia serrata*, de *Centipeda cunnighami*, d'*Helianthus annuus*, de *Cola nitida*, de clou de girofle et de *Bacopa moniera* ; les sels de l'acide salicylique et en particulier le salicylate de zinc ; les extraits d'algues, en particulier de *Laminaria saccharina* ; l'huile de Canola, de Tamanu, de Calophillum, les huiles insaturées en oméga 3 telles que les huiles de rosier muscat, de cassis, d'ecchium, de poisson ; l'α-bisabolol et les extraits de camomille ; l'allantoine ; le diesterphosphorique de vitamine E et C ; la capryloyl glycine ; les tocotrienols ; le piperonal ; l'*Aloe vera* ; les phytostérols ; la cortisone, l'hydrocortisone, l'indometacine la béta méthasone.

On peut citer en outre les antagonistes de substances P et notamment : les sels de strontium ; les eaux thermales et en particulier l'eau thermale du bassin de Vichy et l'eau thermale de La Roche Posay ; les extraits bactériens et en particulier l'extrait de bactéries filamenteuses non photosynthétiques décrit dans la demande de brevet EP-0 761 204, préparé de préférence à partir de bactéries appartenant à l'ordre des Beggiatoales, et plus particulièrement au genre Vitreoscilla. Préférentiellement, on utilise selon l'invention une souche de *Vitreoscilla filiformis.*

On peut également citer les antagonistes de CGRP et en particulier un extrait de cellules (de préférence indifférenciées) d'au moins un végétal de la famille des Iridacées, obtenu par culture *in vitro.* L'Iridacée appartient de préférence au genre Iris. En particulier, on préfère utiliser un extrait aqueux *d'Iris pallida,* comme décrit dans la demande EP-0 765 668.

On peut enfin citer les antagonistes de bradykinine et en particulier un extrait d'au moins un végétal de la famille des Rosacées, de préférence cultivé *in vivo.* Préférentiellement, on utilise selon l'invention un végétal appartenant au genre Rosa, avantageusement de l'espèce Rosa *gallica*, plus préférentiellement un extrait hydroglycolique de pétales de Rosa *gallica*, comme décrit dans la demande de brevet EP-0 909 556.

### Agents anti-bactériens

Les agents antibactériens susceptibles d'être utilisés dans la présente invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophényl imidazol dioxolan et ses dérivés décrits dans le brevet WO9318743, le farnesol, les phytosphingosines et leurs mélanges.

Les agents antibactériens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

L'invention sera maintenant illustrée par les exemples non limitatifs suivants, qui font référence aux Figures 1 à 3 annexées sur lesquelles sont représentés les procédés de préparation des composés mentionnés dans ces exemples.

### EXEMPLES

### Exemple 1 : Synthèse de la 4-méthoxy-2-phényl-1,2-benzisothiazol-3(2H)-one

### Première étape

Comme illustré à la Figure 1, on place 10 g de salicylanilide (0.0469 mol, 1 eq) puis 12.96 g de carbonate de potassium (0.0938 mol, 2 eq) dans un tricol de 500 ml. On ajoute 235 ml d'acétone. On ajoute goutte à goutte 3.51 ml d'iodométhane (0.0563 mol, 1.2 eq). On laisse agiter à température ambiante pendant 18 heures.

Le précipité blanc formé est filtré et le filtrat évaporé sous pression réduite (P = 200 mbar, T = 40°C). L'huile obtenue est reprise dans 150ml d'acétate d'éthyle et lavée par 3x50 ml d'eau et 50 ml de solution aqueuse de chlorure de sodium saturée. La phase organique est séchée sur sulfate de sodium, filtrée et évaporée sous pression réduite (P = 220 mbar, T = 40°C).

On sèche sous vide en présence de di- phosphore pentaoxyde pour obtenir 9.8 g de 2-méthoxy-N-phénylbenzamide **(1a),** sous forme de cire blanche.

### Deuxième étape

Dans un tricol de 500 ml préalablement séché à l'étuve (T= 115°C) et mis sous argon, on place 6 g de **(1a)** 2-méthoxy-N-phénylbenzamide protégé (0.026 mol, 1 eq). On ajoute 180 ml de tétrahydrofurane anhydre puis on refroidit cette solution à 0°C. On ajoute goutte à goutte 30.2 ml de n-butyl lithium titré à 1.75 mol/l (0.052 mol, 2 eq). On laisse agiter à 0°C pendant 45 minutes puis on ajoute 0.846 g de soufre sublimé (0.026 mol, 1 eq). On poursuit l'agitation à 0°C pendant 30 minutes puis le mélange réactionnel est amené à T = -56°C à l'aide d'un bain acétone + carboglace. On ajoute 11.6 g de bromure de cuivre anhydre (0.052 mol, 2 eq). On laisse agiter à -56°C pendant 30 minutes puis à température ambiante pendant 18 heures.

On verse le mélange réactionnel dans 150 ml d'une solution aqueuse de chlorure d'ammonium saturée. Après filtration sur celite 545, on extrait par 3x150 ml d'acétate d'éthyle puis les phases organiques sont lavées avec 100 ml d'une solution aqueuse de chlorure de sodium saturée. La phase organique est séchée sur sulfate de magnésium, filtrée, puis évaporée sous pression réduite (P = 220 mbar, 40 °C).

Le composé (1b) ou 4-méthoxy-2-phényl-1,2-benzisothiazol-3(2H)-one (m = 1.8 g) est obtenu par chromatographie sur colonne de gel de silice (éluant : cyclohexane/acétate d'éthyle : 7/3), sous forme de solide blanc. Les analyses par RMN ¹H et spectrométrie de masse sont conformes à la structure attendue.

Remarque : Le composé disulfure est également isolé et pourra être engagé dans l'étape de S- oxydation en sulfonamide.

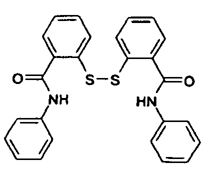

### Exemple 2 : synthèse du 4-méthoxy-2-phényl-1,2-benzisothiazol-3(2H)-one 1,1-dioxide

Comme illustré à la Figure 1, on introduit dans un ballon de 100 ml, 1.15 g de composé (1 b) (0.0045 mol, 1 eq- pureté à 80 %) obtenu à l'Exemple 1 ci-dessus, puis 17.1 ml d'acide trifluoroacétique (0.223 mol, 50 eq) et 1.86 ml de peroxyde d'hydrogène à 30% (0.0178 mol, 4 eq). On laisse agiter à température ambiante pendant 18 heures.

Le milieu réactionnel est dilué par 80 ml de dichlorométhane et lavé par 2 x 80 ml d'eau, 2 x 50 ml d'une solution aqueuse de bicarbonate de sodium saturée, 50 ml d'eau puis 50 ml d'une solution aqueuse de chlorure de sodium saturée. La phase organique est séchée sur sulfate de magnésium, filtrée et évaporée sous pression réduite (P = 220 mbar, T = 40°C).

On obtient 0.83 g de composé (1c) ou 4-méthoxy-2-phényl-1,2-benzisothiazol-3(2H)-one 1,1-dioxide, sous forme de solide beige, après séchage sous pression réduite (P=10⁻¹ mbar, température ambiante). Les analyses par RMN ¹H et spectrométrie de masse sont conformes à la structure attendue.

### Exemple 3 : synthèse du 4-méthoxy-2-phényl-1,2-benzisothiazol-3(2H)-one 1-oxide

Comme illustré à la Figure 1, on introduit dans un tricol de 250 ml, 0.355 g de N-Chlorosuccinimide (0.0026 mol, 1 eq) et 50 ml de dichlorométhane. On refroidit cette solution à 0°C puis on ajoute goutte à goutte en 45 minutes 0.685 g de composé **(1b)** obtenu à l'Exemple 1 et solubilisé dans 25 ml de dichlorométhane. On laisse remonter le mélange réactionnel à température ambiante et l'agitation est maintenue pendant 3 heures.

On ajoute 100 ml de solution de 8 g d' hydrogénocarbonate de sodium dans l'eau. On agite pendant 1 heure à température ambiante. On lave la phase organique avec 2 x 50 ml d'eau puis 50 mL d'une solution aqueuse de chlorure de sodium saturée. La phase organique est séchée sur sulfate de sodium, filtrée et évaporée sous pression réduite (P = 220 mbar, T = 40°C).

On obtient 0.7g de composé (1d) ou 4-méthoxy-2-phényl-1,2-benzisothiazol-3(2H)-one 1-oxide, sous forme de solide beige, après séchage sous pression réduite (P=10-1 mbar, TA). Les analyses par RMN ¹H et spectrométrie de masse sont conformes à la structure attendue.

### Exemple 4 : synthèse du 4-hydroxy-2-phényl-1,2-benzisothiazol-3(2H)-one 1,1-dioxide

Comme illustré à la Figure 1, dans un tricol de 100 ml séché à l'étuve (T = 115°C) puis mis sous argon, on introduit 0.5 g de composé (1c) obtenu à l'Exemple 2. On ajoute 50 ml de dichlorométhane anhydre puis le mélange réactionnel est refroidi à 0°C. On ajoute goutte à goutte le tribromure de bore en solution dans le dichlorométhane à 1M. On laisse agiter à 0°C pendant 3h.

On verse ensuite le milieu réactionnel dans 100 ml d'eau, et l'agitation est maintenue pendant 30 minutes. On lave la phase organique avec 2 x 50 ml d'une solution aqueuse de bicarbonate de sodium saturée puis 2 x 50 ml d'une solution aqueuse de chlorure de sodium saturée. La phase organique est séchée sur sulfate de sodium, filtrée et évaporée sous pression réduite (P = 220 mbar, T = 40°C) et conduit à l'obtention d'un solide beige. Ce dernier est repris par 20 ml de pentane sous agitation, puis filtré pour obtenir 0.4 g du solide beige (1e) ou 4-hydroxy-2-phényl-1,2-benzisothiazol-3(2H)-one 1,1-dioxide après séchage sous pression réduite (P=10⁻¹ mbar, TA). Les analyses par RMN ¹H et spectrométrie de masse sont conformes à la structure attendue.

### Exemple 5 : synthèse de la 4-méthoxy-2-(4-méthoxyphényl)-1,2-benzisothiazol-3(2H)-one

### Première étape

Comme illustré à la Figure 2, on dissout dans un tricol de 50 ml 5.6 g de chlorure d'acide 2-méthoxy benzoïque (0.033 mol, 1.1 eq) dans 20 ml de dichlorométhane puis on ajoute 4.6 ml de triéthylamine (0.033 mol, 1.1 eq). Le mélange réactionnel est refroidi à 0-5°C. On ajoute goutte à goutte en 15 minutes une solution de 3.7 g de p-anisidine (0.03 mol, 1 eq) dans le dichlorométhane. On laisse agiter 1 heure à 0°C puis à température ambiante pendant 18 heures.

On reprend le mélange dans 100 ml de dichlorométhane, on lave avec 3 x 50 ml d'eau puis 50 ml d'une solution aqueuse de chlorure de sodium saturée. La phase organique est séchée sur sulfate de magnésium, filtrée et évaporée sous pression réduite (P = 220 mbar, T = 40°C).

Le produit attendu est obtenu par cristallisation dans le toluène à - 15 °C pendant 2 heures. On isole 2.8 g de composé **(2a)** ou 2-méthoxy-N-(4-méthoxyphényl)benzamide, sous forme de solide beige, qui est ensuite purifié par chromatographie sur colonne de gel de silice (éluant : cyclohexane/acétate d'éthyle : 1/1). Les analyses par RMN ¹H et spectrométrie de masse sont conformes à la structure attendue.

Deuxième étape : On obtient le composé (2b) 4-methoxy-2-(4-methoxyphenyl)-1,2-benzisothiazol-3(2H)-one (rendement = 20 %), sous forme de liquide jaune, en suivant le mode opératoire décrit dans la deuxième étape de l'Exemple 1 et après purification par chromatographie sur colonne de gel de silice (éluant : cyclohexane/acétate d'éthyle : 1/1). Les analyses par RMN ¹H et spectrométrie de masse sont conformes à la structure attendue.

Remarque : l'une des impuretés isolées est le dimère suivant :

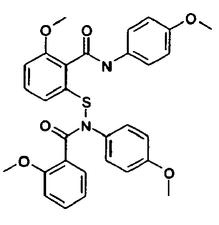

### Exemple 6 : synthèse du 4-méthoxy-2-(4-méthoxyphényl)-1,2-benzisothiazol-3(2H)-one 1,1-dioxide

Comme illustré à la Figure 2, le composé (2b) obtenu comme décrit à l'Exemple 5 peut être mis en oeuvre dans un procédé analogue à celui décrit à l'Exemple 2 pour obtenir le composé **(2c)** ou 4-méthoxy-2-(4-méthoxyphényl)-1,2-benzisothiazol-3(2H)-one 1,1-dioxide, qui peut être isolé sous forme de solide blanc après purification par chromatographie sur colonne de gel de silice (éluant : cyclohexane/acétate d'éthyle : 1/1). Les analyses par RMN ¹H et spectrométrie de masse sont conformes à la structure attendue.

### Exemple 7 : synthèse du 4-méthoxy-2-(4-méthoxyphényl)-1,2-benzisothiazol-3(2H)-one 1-oxide

Comme illustré à la Figure 2, le composé (2b) obtenu comme décrit à l'Exemple 5 peut être mis en oeuvre dans un procédé analogue à celui décrit à l'Exemple 3 pour obtenir le composé (2d) ou 4-méthoxy-2-(4-méthoxyphényl)-1,2-benzisothiazol-3(2H)-one 1-oxide sous forme de solide beige. Les analyses par RMN ¹H et spectrométrie de masse sont conformes à la structure attendue.

### Exemple 8 : synthèse du 4-hydroxy-2-(4-méthoxyphényl)-1,2-benzisothiazol-3(2H)-one 1,1-dioxide

Comme illustré à la Figure 2, le composé (2e) ou 4-hydroxy-2-(4-méthoxyphényl)-1,2-benzisothiazol-3(2H)-one 1,1-dioxide peut être obtenu à partir du composé (2c) obtenu à l'Exemple 6, en mettant en oeuvre un procédé analogue à celui décrit à l'Exemple 4. On obtient un solide beige. Les analyses par RMN ¹H et spectrométrie de masse sont conformes à la structure attendue.

### Exemple 9 : synthèse de la 5,7-diméthoxy-2-phényl-1,2-benzisothiazol-3(2H)-one

### Première étape

Comme illustré à la Figure 3, dans un tricol de 250 ml, sous atmosphère inerte, on introduit 6.3 g de chlorure de 3,5-diméthoxybenzoyle (0.031 mol, 1 eq) dans 60 ml de dichlorométhane anhydre. On ajoute goutte à goutte 9.6 ml de triéthylamine (0.068 mol, 2.2 eq). Le mélange réactionnel est refroidi à 0-5°C. On ajoute progressivement 4.05 g d'aniline.HCl (0.031 mol, 1 eq) en 30 minutes. On laisse agiter 1 heure à 0°C puis à température ambiante pendant 18 heures.

Le mélange réactionnel est repris dans 150 ml de dichlorométhane puis lavé avec 2 x 100 ml d'eau, 100 ml d'une solution d'acide chlorhydrique 0.5 N, 2 x 100 ml d'eau puis 100 ml d'une solution aqueuse de chlorure de sodium saturée. La phase organique est séchée sur sulfate de sodium, filtrée et évaporée sous pression réduite (P = 220 mbar, T = 40°C).

On obtient 4.5 g de composé **(3a)** ou 3,5-diméthoxy-N-phénylbenzamide, sous forme de solide blanc. Les analyses par RMN ¹H et spectrométrie de masse sont conformes à la structure attendue.

### Deuxième étape

On suit le mode opératoire décrit à la deuxième étape de l'Exemple 1 pour former et isoler le composé (3b) ou 5,7-diméthoxy-2-phényl-1,2-benzisothiazol-3(2H)-one à partir du composé **(3a)** obtenu dans la première étape ci-dessus. On obtient un solide blanc. Les analyses par RMN ¹H et spectrométrie de masse sont conformes à la structure attendue.

### Exemple 10 : synthèse du 5,7-diméthoxy-2-phényl-1,2-benzisothiazol-3(2H)-one 1,1-dioxide

Comme illustré à la Figure 3, en présence d'acide trifluoroacétique et de peroxyde d'hydrogène à 30 %, suivant le mode opératoire décrit dans l'Exemple 2, on forme le produit **(3c)** ou 5,7-diméthoxy-2-phényl-1,2-benzisothiazol-3(2H)-one 1,1-dioxide, en mélange avec le composé sulfinamide correspondant **(3d)** en proportion 70/30.

Le composé (3c) peut être purifié par chromatographie sur colonne de gel de silice (éluant : cyclohexane/acétate d'éthyle : 8/2). On obtient un solide blanc. Les analyses par RMN ¹H et spectrométrie de masse sont conformes à la structure attendue.

### Exemple 11 : synthèse du 5,7-diméthoxy-2-phényl-1,2-benzisothiazol-3(2H)-one 1-oxide

Comme illustré à la Figure 3, dans un bicol de 50 ml, sec et sous N₂ , on solubilise dans 2 ml de dichlorométhane 0.2 g de 5,7-diméthoxy-2-phényl-1,2-benzisothiazol-3(2H)-one (0.0007 mol, 1 eq) (3b) obtenue à l'Exemple 9, puis on ajoute 10 ml de méthanol. Le milieu réactionnel est refroidi à 0°C. On ajoute 0.172 g de monoperoxyphtalate de magnésium hexahydrate technique à 80 % (0.00035 mol, 0.5 eq). L'agitation est maintenue à 0°C pendant 3 heures puis à température ambiante pendant 18 heures.

Pour consommer totalement le réactif (3b), on refroidit à nouveau le milieu réactionnel à 0°C, on ajoute 0.5 eq supplémentaire de monoperoxyphtalate de magnésium hexahydrate technique à 80 %. L'agitation est maintenue à 0°C pendant 30 minutes puis on laisse remonter à température ambiante.

On filtre le précipité blanc, puis le filtrat est concentré sous pression réduite (P = 220 mbar, T = 40°C). On reprend ce brut réactionnel dans 50 ml de dichlorométhane puis on lave avec 25 ml d'eau, 25 ml d'une solution aqueuse de bicarbonate de sodium saturée puis 3 x 25 ml d'eau. La phase organique est séchée sur sulfate de sodium, filtrée et évaporée sous pression réduite (P = 220 mbar, T = 40°C).

Après purification par chromatographie sur colonne de gel de silice (éluant : cyclohexane/acétate d'éthyle : 7/3), on isole 0.164 g de composé (3d) ou 5,7-diméthoxy-2-phényl-1,2-benzisothiazol-3(2H)-one 1-oxide, sous forme de solide blanc. Les analyses par RMN ¹H et spectrométrie de masse sont conformes à la structure attendue.

### Exemple 12 : Mise en évidence de l'activité des composés de formule (I) sur la lipogénèse

On a testé l'activité des composés (2c) et (1e) décrits précédemment, sur un modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020 (1999).

Le test a consisté à mesurer la quantité de lipides produite par les sébocytes de la lignée (à confluence), en présence ou non d'agent actif, dilué dans le DMSO, de telle sorte que la quantité finale de DMSO dans le milieu de culture soit 0,1 %. Après 2 jours de traitement, les cellules adhérentes sont traitées par du Rouge de Nile (1µg/ml). Le contenu en lipides est ensuite quantifié par mesure de la fluorescence du colorant (couple d'excitation/émission : 485-540nm pour les lipides neutres).

Les tests sont réalisés en sixplicates et l'expérience est renouvelée quatre fois.

En parallèle, des tests de prolifération (MUH) et de viabilité cellulaire (LDH) permettent de vérifier que les effets obtenus ne sont pas liés à une modification notable de ces paramètres biologiques.

Les résultats sont rassemblés dans le Tableau ci-dessous :

| COMPOSE TESTE | CONCENTRATION | VARIATION DES LIPIDES (par rapport au témoin) | Prolifération / Cytotoxicité |
|---|---|---|---|
| Composé **(2c)** | 10⁻⁶ M | + 43 % | non |
| Composé **(1e)** | 10⁻⁶ M | + 16 % | non |
| | 10⁻⁴ M | + 39 % | non |

Comme il ressort de ce Tableau, les composés selon l'invention induisent une augmentation significative de la lipogénèse sébocytaire.

En outre, dans le même test, la DHEA, testée à la concentration de 10⁻⁵ M n'a conduit qu'à une augmentation de 13 % du contenu en lipides du sébum. Les composés selon l'invention sont donc plus efficaces que la DHEA.

### Exemple 13 : Composition cosmétique (émulsion H/E)

Cette composition est préparée de manière classique pour l'homme du métier. Les quantités données dans cet exemple sont indiquées en pourcentages pondéraux.

| | |
|---|---|
| 4-méthoxy-2-(4-méthoxyphényl)-1,2-benzisothiazol-3(2H)-one 1,1-dioxide | 0.3 % |
| Stéarate de glycérol | 2 % |
| Polysorbate 60 | 1 % |
| Acide stéarique | 1.4 % |
| Triéthanolamine | 0.7 % |
| Carbomer | 0.4 % |
| Huile d'olive | 12 % |
| Fraction liquide du beurre de karité | 12 % |
| Octyldodécanol | 6 % |
| Isononanoate d'isononyle | 10 % |
| Antioxydant | 0.05 % |
| Parfum | 0.5 % |
| Conservateur | 0.3 % |
| Eau qsp | 100 % |

Cette composition, utilisée en applications bi-quotidiennes, permet de redynamiser la fonction sébacée des peaux sèches.

## Revendications

1. Composition, adaptée à une application topique sur la peau, comprenant au moins un composé de formule (I) : 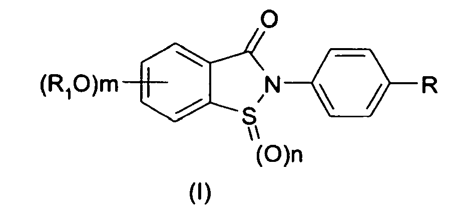 dans laquelle :
• R₁ est un atome d'hydrogène ou un radical méthyle,
• R est un atome d'hydrogène ou un radical méthoxy,
• m est égal à 1 ou 2,
• n est égal à 0, 1 ou 2.

2. Composition selon la revendication 1, **caractérisée en ce que** m est égal à 1.

3. Composition selon la revendication 1 ou 2, **caractérisée en ce que** n est égal à 2.

4. Composition selon la revendication 1, **caractérisée en ce que** le composé de formule (I) est choisi parmi :
• la 4-hydroxy-2-phényl-1,2-benzisothiazol-3(2H)-one
• le 4-hydroxy-2-phényl-1,2-benzisothiazol-3(2H)-one 1-oxide
• le 4-hydroxy-2-phényl-1,2-benzisothiazol-3(2H)-one 1,1-dioxide
• la 4-méthoxy-2-phényl-1,2-benzisothiazol-3(2H)-one
• le 4-méthoxy-2-phényl-1,2-benzisothiazol-3(2H)-one 1-oxide
• le 4-méthoxy-2-phényl-1,2-benzisothiazol-3(2H)-one 1,1-dioxide
• la 4-hydroxy-2-(4-méthoxyphényl)-1,2-benzisothiazol-3(2H)-one
• le 4-hydroxy-2-(4-méthoxyphényl)-1,2-benzisothiazol-3(2H)-one 1-oxide
• le 4-hydroxy-2-(4-méthoxyphényl)-1,2-benzisothiazol-3(2H)-one 1,1-dioxide
• la 4-méthoxy-2-(4-méthoxyphényl)-1,2-benzisothiazol-3(2H)-one
• le 4-méthoxy-2-(4-méthoxyphényl)-1,2-benzisothiazol-3(2H)-one 1-oxide
• le 4-méthoxy-2-(4-méthoxyphényl)-1,2-benzisothiazol-3(2H)-one 1,1-dioxide
• la 5,7-dihydroxy-2-phényl-1,2-benzisothiazol-3(2H)-one
• le 5,7-dihydroxy-2-phényl-1,2-benzisothiazol-3(2H)-one 1-oxide
• le 5,7-dihydroxy-2-phényl-1,2-benzisothiazol-3(2H)-one 1,1-dioxide
• la 5,7-diméthoxy-2-phényl-1,2-benzisothiazol-3(2H)-one
• le 5,7-diméthoxy-2-phényl-1,2-benzisothiazol-3(2H)-one 1-oxide
• le 5,7-diméthoxy-2-phényl-1,2-benzisothiazol-3(2H)-one 1,1-dioxide
• la 5,7-dihydroxy-2-(4-méthoxyphényl)-1,2-benzisothiazol-3(2H)-one
• le 5,7-dihydroxy-2-(4-méthoxyphényl)-1,2-benzisothiazol-3(2H)-one 1-oxide
• le 5,7-dihydroxy-2-(4-méthoxyphényl)-1,2-benzisothiazol-3(2H)-one 1,1-dioxide
• la 5,7-diméthoxy-2-(4-méthoxyphényl)-1,2-benzisothiazol-3(2H)-one
• le 5,7-diméthoxy-2-(4-méthoxyphényl)-1,2-benzisothiazol-3(2H)-one 1-oxide
• le 5,7-diméthoxy-2-(4-méthoxyphényl)-1,2-benzisothiazol-3(2H)-one 1,1-dioxide

5. Composition selon l'une quelconque des revendications 1 à 4, **caractérisée en ce que** le composé de formule (I) est le 4-méthoxy-2-(4-méthoxyphényl)-1,2-benzisothiazol-3(2H)-one 1,1-dioxide.

6. Composition selon l'une quelconque des revendications 1 à 4, **caractérisée en ce que** le composé de formule (I) est le 4-hydroxy-2-phényl-1,2-benzisothiazol-3(2H)-one 1,1-dioxide.

7. Composition selon l'une quelconque des revendications 1 à 6, **caractérisée en ce qu'**elle renferme un milieu cosmétiquement acceptable.

8. Composition selon l'une quelconque des revendications 1 à 7, **caractérisée en ce qu'**elle renferme en outre au moins un composé choisi parmi : un agent desquamant, un agent hydratant, un agent apaisant, un agent favorisant la prolifération et/ou la différenciation des kératinocytes et un agent anti-bactérien.

9. Composition selon l'une quelconque des revendications 1 à 8, **caractérisée en ce qu'**elle se présente sous la forme d'une émulsion huile-dans-eau.

10. Benzoisothiazolones, **caractérisées en ce qu'**elles sont choisies parmi les composés de formule (I) tels que définis dans l'une quelconque des revendications 1 à 6, à l'exception de ceux pour lesquels :
• n=0;R=H;m=1; et R₁=CH₃ ; et
• n = 2 ; R = H ; m = 1 ; et R₁ = CH₃.

11. Procédé cosmétique de soin d'une peau sèche et/ou mature, comprenant l'application topique sur la peau, en particulier du visage et/ou du cou et/ou des mains, de la composition selon l'une quelconque des revendications 1 à 9.

12. Procédé selon la revendication 11, **caractérisé en ce que** la composition est appliquée sur des femmes de plus de quarante ans.

13. Utilisation d'au moins un composé de formule (I), tel que défini dans l'une quelconque des revendications 1 à 6 ou dans la revendication 10, pour la préparation d'une composition dermatologique destinée à traiter les dermites.

## Claims

1. Composition, suitable for topical application to the skin, comprising at least one compound of formula (I): 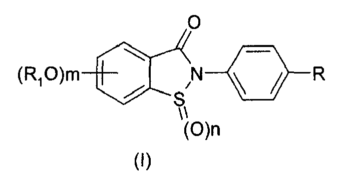 in which:
• R₁ is a hydrogen atom or a methyl radical,
• R is a hydrogen atom or a methoxy radical,
• m is equal to 1 or 2,
• n is equal to 0, 1 or 2.

2. Composition according to Claim 1, **characterized in that** m is equal to 1.

3. Composition according to Claim 1 or 2, **characterized in that** n is equal to 2.

4. Composition according to Claim 1, **characterized in that** the compound of formula (I) is chosen from:
• 4-hydroxy-2-phenyl-1,2-benzisothiazol-3(2H)-one
• 4-hydroxy-2-phenyl-1,2-benzisothiazol-3(2H)-one 1-oxide
• 4-hydroxy-2-phenyl-1,2-benzisothiazol-3(2H)-one 1,1-dioxide
• 4-methoxy-2-phenyl-1,2-benzisothiazol-3(2H)-one
• 4-methoxy-2-phenyl-1,2-benzisothiazol-3(2H)-one 1-oxide
• 4-methoxy-2-phenyl-1,2-benzisothiazol-3(2H)-one 1,1-dioxide
• 4-hydroxy-2-(4-methoxyphenyl)-1,2-benzisothiazol-3(2H)-one
• 4-hydroxy-2-(4-methoxyphenyl)-1,2-benzisothiazol-3(2H)-one 1-oxide
• 4-hydroxy-2-(4-methoxyphenyl)-1,2-benzisothiazol-3(2H)-one 1,1-dioxide
• 4-methoxy-2-(4-methoxyphenyl)-1,2-benzisothiazol-3(2H)-one
• 4-methoxy-2-(4-methoxyphenyl)-1,2-benzisothiazol-3(2H)-one 1-oxide
• 4-methoxy-2-(4-methoxyphenyl)-1,2-benzisothiazol-3(2H)-one 1,1-dioxide
• 5,7-dihydroxy-2-phenyl-1,2-benzisothiazol-3(2H)-one
• 5,7-dihydroxy-2-phenyl-1,2-benzisothiazol-3(2H)-one 1-oxide
• 5,7-dihydroxy-2-phenyl-1,2-benzisothiazol-3(2H)-one 1,1-dioxide
• 5,7-dimethoxy-2-phenyl-1,2-benzisothiazol-3(2H)-one
• 5,7-dimethoxy-2-phenyl-1,2-benzisothiazol-3(2H)-one 1-oxide
• 5,7-dimethoxy-2-phenyl-1,2-benzisothiazol-3(2H)-one 1,1-dioxide
• 5,7-dihydroxy-2-(4-methoxyphenyl)-1,2-benzisothiazol-3(2H)-one
• 5,7-dihydroxy-2-(4-methoxyphenyl)-1,2-benzisothiazol-3(2H)-one 1-oxide
• 5,7-dihydroxy-2-(4-methoxyphenyl)-1,2-benzisothiazol-3(2H)-one 1,1-dioxide
• 5,7-dimethoxy-2-(4-methoxyphenyl)-1,2-benzisothiazol-3(2H)-one
• 5,7-dimethoxy-2-(4-methoxyphenyl)-1,2-benzisothiazol-3(2H)-one 1-oxide
• 5,7-dimethoxy-2-(4-methoxyphenyl)-1,2-benzisothiazol-3(2H)-one 1,1-dioxide.

5. Composition according to any one of Claims 1 to 4, **characterized in that** the compound of formula (I) is 4-methoxy-2-(4-methoxyphenyl)-1,2-benzisothiazol-3(2H)-one 1,1-dioxide.

6. Composition according to any one of Claims 1 to 4, **characterized in that** the compound of formula (I) is 4-hydroxy-2-phenyl-1,2-benzisothiazol-3(2H)-one 1,1-dioxide.

7. Composition according to any one of Claims 1 to 6, **characterized in that** it contains a cosmetically acceptable medium.

8. Composition according to any one of Claims 1 to 7, **characterized in that** it also contains at least one compound chosen from: a desquamating agent, a moisturizer, a calmative agent, an agent for promoting keratinocyte proliferation and/or differentiation, and an antibacterial agent.

9. Composition according to any one of Claims 1 to 8, **characterized in that** it is in the form of an oil-in-water emulsion.

10. Benzoisothiazolones, **characterized in that** they are chosen from the compounds of formula (I) as defined in any one of Claims 1 to 6, with the exception of those for which:
• n = 0; R = H; m = 1; and R₁ = CH₃ ; and
• n = 2; R = H; m = 1; and R₁ = CH₃.

11. Cosmetic method of caring for dry and/or mature skin, comprising the topical application of the composition according to any one of Claims 1 to 9 to the skin, in particular of the face and/or of the neck and/or of the hands.

12. Method according to Claim 11, **characterized in that** the composition is applied to women over the age of forty.

13. Use of at least one compound of formula (I) as defined in any one of Claims 1 to 6 or in Claim 10, for preparing a dermatological composition for treating forms of dermatitis.

## Patentansprüche

1. Zusammensetzung, die für eine topische Anwendung auf die Haut geeignet ist und die mindestens eine Verbindung der folgenden Formel (I) enthält: 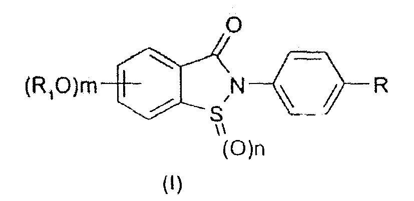 in der Formel:
· R₁ bedeutet ein Wasserstoffatom oder die Methylgruppe;
· R bedeutet ein Wasserstoffatom oder die Methoxygruppe;
· m ist 1 oder 2;
· n ist 0, 1 oder 2.

2. Zusammensetzung nach Anspruch 1, **dadurch gekennzeichnet, dass** m 1 bedeutet.

3. Zusammensetzung nach Anspruch 1 oder 2, **dadurch gekennzeichnet, dass** n 2 bedeutet.

4. Zusammensetzung nach Anspruch 1, **dadurch gekennzeichnet, dass** die Verbindung der Formel (I) ausgewählt ist unter:
· 4-Hydroxy-2-phenyl-1,2-benzisothiazol-3(2H)-on
· 4-Hydroxy-2-phenyl-1,2-benzisothiazol-3(2H)-on-1-oxid
· 4-Hydroxy-2-phenyl-1,2-benzisothiazol-3(2H)-on-1,1-dioxid
· 4-Methoxy-2-phenyl-1,2-benzisothiazol-3(2H)-on
· 4-Methoxy-2-phenyl-1,2-benzisothiazol-3(2H)-on-1-oxid
. 4-Methoxy-2-phenyl-1,2-benzisothiazol-3(2H)-on-1,1-dioxid
· 4-Hydroxy-2-(4-methoxyphenyl)-1,2-benzisothiazol-3(2H)-on
· 4-Hydroxy-2-(4-methoxyphenyl)-1,2-benzisothiazol-3(2H)-on-1-oxid
· 4-Hydroxy-2-(4-methoxyphenyl)-1,2-benzisothiazol-3(2H)-on-1,1-dioxid
· 4-Methoxy-2-(4-methoxyphenyl)-1,2-benzisothiazol-3(2H)-on
· 4-Methoxy-2-(4-methoxyphenyl)-1,2-benzisothiazol-3(2H)-on-1-oxid
· 4-Methoxy-2-(4-methoxyphenyl)-1,2-benzisothiazol-3(2H)-on-1,1-dioxid
· 5,7-Dihydroxy-2-phenyl-1,2-benzisothiazol-3(2H)-on
· 5,7-Dihydroxy-2-phenyl-1,2-benzisothiazol-3(2H)-on-1-oxid
· 5,7-Dihydroxy-2-phenyl-1,2-benzisothiazol-3(2H)-on-1,1-dioxid
· 5,7-Dimethoxy-2-phenyl-1,2-benzisothiazol-3(2H)-on
· 5,7-Dimethoxy--2-phenyl-1,2-benzisothiazol-3(2H)-on-1-oxid
· 5,7-Dimethoxy-2-phenyl-1,2-benzisothiazol-3(2H)-on-1,1-dioxid
· 5,7-Dihydroxy-2-(4-methoxyphenyl)-1,2-benzisothiazol-3(2H)-on
· 5,7-Dihydroxy-2-(4-methoxyphenyl)-1,2-benzisothiazol-3(2H)-on-1-oxid
· 5,7-Dihydroxy-2-(4-methoxyphenyl)-1,2-benzisothiazol-3(2H)-on-1,1-dioxid
· 5,7-Dimethoxy-2-(4-methoxyphenyl)-1,2-benzisothiazol-3(2H)-on
· 5,7-Dimethoxy-2-(4-methoxyphenyl)-1,2-benzisothiazol-3(2H)-on-1-oxid
· 5,7-Dimethoxy-2-(4-methoxyphenyl)-1,2-benzisothiazol-3(2H)-on-1,1-dioxid

5. Zusammensetzung nach einem der Ansprüche 1 bis 4, **dadurch gekennzeichnet, dass** es sich bei der Verbindung der Formel (I) um das 4-Methoxy-2-(4-methoxyphenyl)-1,2-benzisothiazol-3 (2H)-on-1,1-dioxid handelt.

6. Zusammensetzung nach einem der Ansprüche 1 bis 4, **dadurch gekennzeichnet, dass** es sich bei der Verbindung der Formel (I) um das 4-Hydroxy-2-phenyl-1,2-benzisothiazol-3(2H)-on-1,1-dioxid handelt.

7. Zusammensetzung nach einem der Ansprüche 1 bis 6, **dadurch gekennzeichnet, dass** sie ein kosmetisch akzeptables Medium enthält.

8. Zusammensetzung nach einem der Ansprüche 1 bis 7, **dadurch gekennzeichnet, dass** sie ferner mindestens eine Verbindung enthält, die unter den folgenden Verbindungen ausgewählt ist: abschuppenden Wirkstoffen, Hydratisierungsmitteln, beruhigenden Stoffen, Wirkstoffen, die die Proliferation und/ oder Differenzierung von Keratinocyten fördern, und antibakteriellen Wirkstoffen.

9. Zusammensetzung nach einem der Ansprüche 1 bis 8, **dadurch gekennzeichnet, dass** sie in Form einer Öl-in-Wasser-Emulsion vorliegt.

10. Benzoisothiazolone, **dadurch gekennzeichnet, dass** sie unter den Verbindungen der Formel (I) ausgewählt sind, wie sie in einem der Ansprüche 1 bis 6 definiert sind, mit Ausnahme von Verbindungen mit:
· n = 0; R = H; m = 1; und R₁ = CH₃; und
. n = 2 ; R = H ; m = 1 ; und R₁ = CH₃.

11. Kosmetisches Verfahren für die Pflege trockener und/oder reifer Haut, das das topische Auftragen einer Zusammensetzung nach einem der Ansprüche 1 bis 9 auf die Haut und insbesondere das Gesicht und/oder den Hals und/oder die Hände umfasst.

12. Verfahren nach Anspruch 11, **dadurch gekennzeichnet, dass** die Zusammensetzung von Frauen, die älter als 40 Jahre sind, angewandt wird.

13. Verwendung mindestens einer Verbindung der Formel (I), wie sie in einem der Ansprüche 1 bis 6 oder Anspruch 10 definiert ist, für die Herstellung einer dermatologischen Zusammensetzung, die für die Behandlung von Dermatitis vorgesehen ist.
